# EUROPÄISCHE PATENTSCHRIFT

(11) **EP 1 561 745 B1**
(45) Veröffentlichungstag und Bekanntmachung des Hinweises auf die Patenterteilung: **01.03.2017**
(21) Anmeldenummer: 05001280.6
(22) Anmeldetag: 22.01.2005
(51) Int. Cl.: C07C 263/10, C07C 263/20, C07C 265/14, C08G 18/76

(54) **Herstellung von Di- und Polyisocyanaten der Diphenylmethanreihe mit definierter Acidität**
Process for the preparation of di- and polyisocyanates of the diphenylmethane series with defined acidity
Procédé pour la préparation de di- et polyisocyanates de la série de diphenylmethane avec acidité définie

(30) Priorität: 04.02.2004 DE 102004005320
(43) Veröffentlichungstag der Anmeldung: 10.08.2005
(73) Patentinhaber: Covestro Deutschland AG, 51373 Leverkusen (DE)
(72) Erfinder: Liman, Ulrich, Dr., 40764 Langenfeld (DE); Müller, Heinz-Herbert, Dr., 47809 Krefeld (DE); Vieler, Robert, Dr., 41542 Dormagen (DE); Echterhoff, Ralf, Dr., 41539 Dormagen (DE)
(74) Vertreter: Levpat

(56) Entgegenhaltungen:
- WO-A-99/54289
- WO-A-02/070581
- DE-A1- 1 938 384
- DE-A1- 3 145 010
- DE-B- 1 138 040

## Beschreibung

Die vorliegende Erfindung betrifft ein Verfahren zur Herstellung von Di- und Polyisocyanaten der Diphenylmethanreihe und deren Mischungen, die geeignet sind zur Herstellung von Polyurethanen.

Aromatische Isocyanate sind sehr wichtige Rohstoffe für die Herstellung von Polyurethan-Werkstoffen. Hierbei spielen die Di- und Polyisocyanate der Diphenylmethanreihe (MDI) die mengenmäßig größte Rolle.

Unter Polyisocyanaten der Diphenylmethanreihe werden Isocyanate und Isocyanatgemische folgenden Typs verstanden: 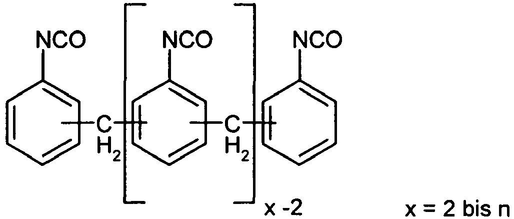 wobei n für eine natürliche Zahl > 0 steht.

Analog werden unter Polyaminen der Diphenylmethanreihe Verbindungen und Verbindungsgemische folgenden Typs verstanden:

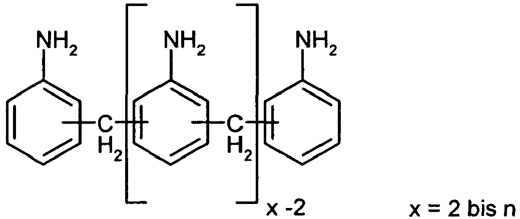

Es ist bekannt, dass Di- und Polyisocyanate der Diphenylmethanreihe (MDI) durch Phosgenierung der entsprechenden Di- und Polyamine der Diphenylmethanreihe (MDA) hergestellt werden. Die Di- und Polyamine der Diphenylmethanreihe selbst werden durch Kondensation von Anilin und Formaldehyd hergestellt. Durch Phosgenierung der Diamine der Diphenylmethanreihe erhält man die entsprechenden Diisocyanate 2,2'-MDI, 2,4'-MDI und 4,4'-MDI, die in der Fachwelt als 2-Kern-MDI (Diisocyanate der Diphenylmethanreihe) beschrieben werden. Während der Kondensation von Anilin und Formaldehyd reagiert das 2-Kern-MDA (Methylendiphenyldiamin MDA) jedoch auch noch weiter mit Formaldehyd und Anilin zu höherkernigen MDA-Typen, die nach der Phosgenierung den Mehrkerngehalt im polymeren MDI (Polyisocyanate der Diphenylmethanreihe) darstellen.

Für viele praktische Produktanwendungen ist es nun bevorzugt, einen definierten Säuregehalt im MDI einzustellen. Diese Standardisierung nivelliert Reaktivitätsschwankungen, die durch Nebenverbindungen in der Reaktionskette vom Anilin zum MDI entstehen. Diese Nebenverbindungen haben einen Einfluss auf den Säuregehalt des MDI bei der Verarbeitung mit H-aktiven Verbindungen wie die in der Polyurethan-Chemie eingesetzten Polyole.

In der Praxis wird der Säuregehalt im MDI als Acidität im polymeren MDI (PMDI) und/oder monomeren MDI (MMDI) oder als hydrolysierbares Chlor im MMDI ausgedrückt. Dieser Aciditäts-Wert wird in der Praxis durch Reaktion von MDI mit niederen Alkoholen, z.B. Methanol, ermittelt (siehe z.B. ASTM D5523-94 für monomeres MDI oder ASTM5629-99 bzw. 6099-03 für polymeres MDI).

Der Säuregehalt im MDI wird nach dem derzeitigen Stand der Technik durch die Prozessverfahrensparameter bei der Herstellung der MDA-Basen durch die Bildung von Nebenkomponenten im MDA, die von den jeweiligen MDA-Prozessparametern abhängt, und bei der Phosgenierung und der anschließenden Aufarbeitung bestimmt. Aufgrund der mehrstufigen Reaktionskette und der Schwankungen und Änderungen des Durchsatzes sowie der Prozessparameter während der Herstellung führt dies in der Praxis zu unvermeidbaren Schwankungen im Säuregehalt. Dabei werden üblicherweise Werte für den Säuregehalt im MDI als Acidität von MDI von bis zu 500 ppm Säure (berechnet als HCl) erreicht. Dabei ergeben sich jedoch mitunter Abweichungen von der gewünschten Acidität. Diese Schwankungen in der Acidität innerhalb einer hergestellten MDI-Produktqualität führen zu unerwünschten Abweichungen im Reaktionsverhalten des MDI bei der späteren Herstellung der Polyurethane.

DE-A-1 138 040 befasst sich mit einem Verfahren zur Entfernung von hydroylsierbarem Chlor und Verringerung der Acidität aus bzw. in organischen Isocyanaten (vgl. Titel, Patentanspruch 1).

DE 31 45 010 A1 befasst sich mit einem Verfahren zur Herstellung von hochreinem 4,4'-MDI durch Einhaltung bestimmter Destillationsbedingungen (siehe Zusammenfassung, Patentanspruch 1).

WO 02/070581 A1 befasst sich mit einem Verfahren zur Bereitstellung von 2,4'-MDI ohne die Qualität des Gesamtverfahrens und die Zusammensetzung der übrigen MDI-Produkte nachteilig zu beeinflussen (S. 3, Z. 32 bis 40). Die Standardisierung von MDI-Partien bzgl. ihres Säuregehalts ist nicht Gegenstand des in WO 02/070581 A1 beschriebenen Verfahrens.

DE 1 938 384 befasst sich mit einem Verfahren zur Verringerung des Anteils an sauren, Chlor enthaltenden Verbindungen in MDI durch Schmelzkristallisation (vgl. Titel, Patentanspruch 1).

Generell gilt, dass sich bei steigendem Säuregehalt die Reaktivität des MDI zum Polyol verlangsamt. Da jedoch bei der Polyurethan-Verarbeitung die Reaktivität in der Regel durch die Katalyse im Polyol definiert werden soll, ist es essentiell für eine prozesssichere und reproduzierbare Verarbeitung, dass die MDI Komponente eine konstante Reaktivität hat. Der bei der Herstellung nach dem Stand der Technik erreichte Säuregehalt (insbesondere beim MMDI) kann sogar so niedrig sein, dass ungewünschte Nebenreaktionen bei der Polyurethan-Verarbeitung auftreten (z.B. alkalisch katalysierte Polyisocyanaurat Reaktion).

Im Markt werden daher die verschiedenen gehandelten MDI-Produkte mit einem Spezifikationswert für die Acidität bzw. dem Gehalt an hydrolisierbarem Chlor (HC-Wert) spezifiziert. Dabei werden üblicherweise die Analysen der Acidität gemäß den oben genannten Testmethoden ASTM durchgeführt, die den Gehalt an freigesetzter Säure bei Umsetzung mit H-aktiven Verbindungen beschreiben. In der Regel wird dieser Wert als HCl berechnet.

Die Aufgabe der vorliegenden Erfindung ist daher, ein einfaches Verfahren zur Herstellung von Di- und Polyisocyanaten der Diphenylmethanreihe und Gemischen daraus mit definiertem und konstantem Wert für den Säuregehalt (Acidität) bereit zustellen, mit dem eine konstante MDI-Produktqualität zuverlässig gewährleistet werden kann.

Die Erfindung betrifft ein Verfahren zur Herstellung von Di- und Polyisocyanaten der Diphenylmethanreihe und Gemischen daraus, bei dem man
a) Anilin und Formaldehyd in Gegenwart eines sauren Katalysators zu Di- und Polyaminen der Diphenylmethanreihe umsetzt, und
b) die Di- und Polyamine der Diphenylmethanreihe gegebenenfalls in Gegenwart eines Lösungsmittels phosgeniert, wobei man ein rohes Di- und Polyisocyanat erhält, und
c) aus dem rohen Di- und Polyisocyanat destillativ wenigstens eine Fraktion enthaltend mindestens 5 Gew.-% 3-Kern- und höherkerniges MDI sowie wenigstens eine Fraktion enthaltend mindestens 95 Gew.-% 2-Kern-MDI abtrennt, und
d) die Acidität der Fraktion enthaltend mindestens 5 Gew.-% 3-Kern- und höherkerniges MDI analytisch bestimmt, und
e) die Fraktion enthaltend mindestens 5 Gew.-% 3-Kern- und höherkerniges MDI mit einer sauren Verbindung versetzt, so dass die gewünschte Acidität gezielt eingestellt wird, wobei zusätzlich eine inerte Übergangsmetallverbindung enthaltend Eisen zugesetzt wird,
wobei die saure Verbindung ausgewählt ist aus organischen Säuren, wasserfreien Mineralsäuren, anorganischen wasserfreien Lewis-Säuren, aliphatischen und/oder aromatischen und/ oder cycloaliphatischen Carbonsäurehalogeniden, aliphatischen und/oder aromatischen und/oder cycloaliphatischen Carbaminsäurehalogeniden, sauren Chlor-Silanverbindungen, Sulfonsäurehalogeniden und Carbonsäureanhydriden, und wobei die inerte Übergangsmetallverbindung enthaltend Eisen wasserfrei ist und ausgewählt ist aus Fe(III)-Chlorid, Fe(II)-Acetat, Fe(III)-Bromid, Fe(II)-Fluorid, Fe(II)-Iodid, Fe(II)-Fumarat, Fe(III)-tribenzoat, Fe(III)-trisalicylat, Fe(III)-trilaurat, Fe (III)-tripalmitat, Fe(III)-tristearat, Fe(III)-trioleat, Fe-Naphthenate, Fe(II)-2-Ethylhexanoat, Fe(II)-tris(4-cyclohexylbutyrat), Fe(III)-Ethoxid, Fe(III)-i-Propoxid, Fe(III)-Acetylacetonat, Fe(III)-Benzoylacetonat, Fe(II)-Phthalocyanin, Ferrocen und Fe(III)-Trifluoracetylacetonat.

Anschließend werden die in Schritt e) erzeugten Fraktionen bevorzugt in Behälter abgefüllt und stehen dann als Isocyanatkomponente mit exakt eingestellter Acidität für die Herstellung von modifizierten Isocyanaten, Abmischprodukten mit anderen Isocyanatfraktionen, Prepolymeren und Polyurethanen zur Verfügung.

Durch die Zugabe der sauren organischen oder anorganischen Verbindungen als Additiv zum MDI in Schritt e) wird erreicht, dass die spezifizierten Werte des MDI unabhängig vom Herstellprozess exakt und reproduzierbar eingestellt werden können.

Die Erfindung betrifft somit ein Verfahren zur Herstellung von MDI und deren Gemischen, bei dem die Acidität des MDI bzw. deren Gemische durch Zugabe saurer, insbesondere halogenhaltiger und vor allem chlorhaltiger Verbindungen nach der destillativen Auftrennung des rohen MDI-Gemisches in die Isomeren bzw. Isomerengemische gezielt eingestellt wird. Dabei wird durch die gezielte Einstellung der Acidität eine konstante Reaktivität des MDI gewährleistet, ohne die bisher vorhandenen Produktspezifikationen zu verletzen.

Das im erfindungsgemäßen Verfahren eingesetzte Polyamin bzw. Polyamingemisch der Diphenylmethanreihe wird in Schritt a) durch Kondensation von Anilin und Formaldehyd in Gegenwart eines sauren Katalysators erhalten (H. J. Twitchett, Chem. Soc. Rev. 3(2), 209 (1974); W. M. Moore in: Kirk-Othmer Encycl. Chem. Technol., 3rd. Ed., New York, 2, 338-348 (1978)). Für das erfindungsgemäße Verfahren ist es dabei unerheblich, ob Anilin und Formaldehyd zunächst in Abwesenheit des sauren Katalysators vermischt werden und der saure Katalysator anschließend zugesetzt wird oder ob ein Gemisch aus Anilin und saurem Katalysator mit Formaldehyd zu Reaktion gebracht wird.

Geeignete Polyamingemische der Diphenylmethanreihe werden üblicherweise erhalten durch Kondensation von Anilin und Formaldehyd im Stoffmengenverhältnis 20 bis 1,6, bevorzugt 10 bis 1,8 sowie einem Stoffmengenverhältnis von Anilin und saurem Katalysator von 20 bis 1, bevorzugt 10 bis 2.

Formaldehyd wird technisch üblicherweise als wässrige Lösung eingesetzt. Hierbei kann der Wassergehalt von 1 bis 95% variieren. Bevorzugt wird eine wässrige Lösung mit 50 bis 80% Wasser eingesetzt. Es können jedoch auch andere methylengruppenliefernde Verbindungen wie z.B. Polyoxymethylenglykol, *para*-Formaldehyd oder Trioxan eingesetzt werden.

Als saure Katalysatoren haben sich starke organische und vorzugsweise anorganische Säuren bewährt. Geeignete Säuren sind beispielsweise Salzsäure, Schwefelsäure, Phosphorsäure, und Methansulfonsäure. Bevorzugt wird Salzsäure eingesetzt.

In einer bevorzugten Ausführungsform des Verfahrens wird Anilin und saurer Katalysator zunächst vermischt. Dieses Gemisch wird, ggf. nach Abfuhr von Wärme, in einem weiteren Schritt mit Formaldehyd bei Temperaturen zwischen 20°C und 100°C, bevorzugt bei 30°C bis 70°C in geeigneter Weise vermischt und anschließend in einem geeigneten Verweilzeitapparat einer Vorreaktion zu unterzogen. Die Vorreaktion erfolgt bei Temperaturen zwischen 20°C bis 100°C, vorzugsweise im Temperaturbereich 30°C bis 80°C. Im Anschluss an Vermischung und Vorreaktion wird die Temperatur des Reaktionsgemisches in Stufen oder kontinuierlich und ggf. unter

Überdruck auf eine Temperatur von 100°C bis 250°C, bevorzugt auf 100°C bis 180°C, besonders bevorzugt auf eine Temperatur von 100°C bis 160°C gebracht.

Es ist jedoch ebenfalls möglich, in einer anderen Ausführungsform des Verfahrens Anilin und Formaldehyd zunächst in Abwesenheit des sauren Katalysators im Temperaturbereich von 5°C bis 130°C, bevorzugt von 40°C bis 100°C, besonders bevorzugt von 60°C bis 90°C, zu vermischen und zur Reaktion zu bringen. Hierbei bilden sich Kondensationsprodukte aus Anilin und Formaldehyd (sog. Aminal). Im Anschluss an die Aminalbildung kann im Reaktionsgemisch vorhandenes Wasser durch Phasentrennung oder andere geeignete Verfahrensschritte, beispielsweise durch Destillation, entfernt werden. Das Kondensationsprodukt wird dann in einem weiteren Verfahrensschritt mit dem sauren Katalysator in geeigneter Weise vermischt und in einem Verweilzeitapparat bei 20°C bis 100°C, bevorzugt 30°C bis 80°C einer Vorreaktion unterzogen. Anschließend wird die Temperatur des Reaktionsgemisches in Stufen oder kontinuierlich und ggf. unter Überdruck auf eine Temperatur von 100°C bis 250°C, bevorzugt auf 100°C bis 180°C, besonders bevorzugt auf eine Temperatur von 100°C bis 160°C gebracht.

Zur Aufarbeitung des sauren Reaktionsgemisches wird das Reaktionsgemisch nach dem Stand der Technik mit einer Base neutralisiert. Nach dem Stand der Technik erfolgt die Neutralisation üblicherweise bei Temperaturen von beispielsweise 90 - 100°C (H.J. Twitchett, Chem. Soc. Rev. 3(2), 223 (1974)). Als Basen sind beispielsweise geeignet die Hydroxide der Alkali- und Erdalkalielemente. Vorzugsweise kommt wässrige NaOH zur Anwendung.

Im Anschluss an die Neutralisation wird nach dem Stand der Technik die organische von der wässrigen Phase durch geeignete Verfahren (z.B. Phasentrennung in einer Scheideflasche) getrennt. Diese Trennung von organischer und wässriger Phase kann bei der selben Temperatur erfolgen, bei der die Neutralisation des sauren Umlagerungsgemisches erfolgte. Die nach Abtrennung der wässrigen Phase verbleibende produktenthaltende organische Phase wird nach dem Stand der Technik zur Abtrennung von Salzen und überschüssiger Base einer Wäsche unterzogen. Anschließend wird die gereinigte organische Phase von überschüssigem Anilin und anderen im Gemisch vorhandenen Stoffen (z.B. weiteren Lösungsmitteln) durch geeignete physikalische Trennverfahren wie z.B. Destillation, Extraktion oder Kristallisation befreit.

Das so erhaltene Polyamin der Diphenylmethanreihe (Roh-MDA) aus Schritt a) wird im Schritt b) nach den bekannten Methoden mit Phosgen in einem inerten organischen Lösungsmittel zu den entsprechenden Isocyanaten umgesetzt. Das Molverhältnis von Roh-MDA zu Phosgen wird zweckmäßigerweise so bemessen, dass pro Mol NH₂-Gruppe 1 bis 10 Mol, vorzugsweise 1,3 bis 4 Mol Phosgen in der Reaktionsmischung vorliegen. Als inerte Lösungsmittel haben sich chlorierte, aromatische Kohlenwasserstoffe wie z.B. Monochlorbenzol, Dichlorbenzole, Trichlorbenzole, die entsprechenden Toluole und Xylole sowie Chlorethylbenzol bewährt. Insbesondere Anwendung finden als inerte organische Lösungsmittel Monochlorbenzol, Dichlorbenzol oder Mischungen dieser Chlorbenzole. Die Menge an Lösungsmittel wird vorzugsweise so bemessen, dass die Reaktionsmischung einen Isocyanatgehalt von 2 bis 40 Gew.-%, vorzugsweise zwischen 5 uns 20 Gew.-%, bezogen auf das Gesamtgewicht der Reaktionsmischung, aufweist. Die Reaktion von Roh-MDA mit Phosgen wird hierbei bei Temperaturen von 50 bis 250°C und bei Drücken von Umgebungsdruck bis hin zu 50 bar durchgeführt. Bevorzugt wird bei 70 bis 180°C und 2 bis 20 bar gearbeitet.

Nach beendeter Phosgenierung werden aus der Reaktionsmischung das überschüssige Phosgen, das inerte organische Lösungsmittel, die gebildete HCl oder Mischungen davon durch geeignete Verfahren (z.B. Destillation) abgetrennt. Hierzu wird der Druck schrittweise bis ins Vakuum reduziert und der verbliebene Phosgenüberschuss und die gebildete HCl verdampft und abgetrennt. Anschließend wird bevorzugt per Verdampfung das Lösungsmittel schrittweise reduziert unter weiterer Reduzierung des absoluten Drucks auf bis zu 1 mbar, bevorzugt auf bis zu 5 mbar. Parallel dazu wird die Temperatur erhöht bis das Lösungsmittel fast vollständig bis auf weit weniger als 0,1% entfernt ist. Letztlich enthält man in diesem Schritt b) ein rohes Di- und Polyisocyanat (rohes MDI-Gemisch).

Die Auftrennung kann destillativ unter Vakuum oder durch Kristallisation erfolgen. Hierbei ist auch eine Kombination beider Prozesse möglich.

In Schritt c) wird aus dem rohen Di- und Polyisocyanat destillativ wenigstens eine Fraktion enthaltend mindestens 5 Gew.-% 3-Kern- und höherkerniges MDI sowie wenigstens eine Fraktion enthaltend mindestens 95 Gew.-% 2-Kern-MDI abgetrennt.

Die Fraktion enthaltend mindestens 5 Gew.-% 3-Kern- und höherkerniges MDI weist dabei bevorzugt eine Acidität von 5 bis 500 ppm (berechnet als HCl) auf. Die Fraktion enthaltend mindestens 95 Gew.-% 2-Kern-MDI weist dabei bevorzugt eine Acidität von 0,1 bis 50 ppm (berechnet als HCl) auf. Den angegebenen Werten für die Acidität liegt dabei die in Beispiel 1 angegebene Methode zur Bestimmung der Acidität zugrunde.

In Schritt d) wird die Acidität der in Schritt c) hergestellten Fraktion enthaltend mindestens 5 Gew.-% 3-Kern- und höherkerniges MDI analytisch bestimmt. Dies kann nach den Vorschriften nach ASTM5629-99 oder 6099-03 für polymeres MDI oder nach der in Beispiel 1 angegebenen Methode erfolgen.

Üblicherweise können Aciditätsschwankungen im PMDI bei einer mittleren Acidität von z.B. ca. 100 ppm im Bereich von bis zu 50% auftreten, die durch Schwankungen oder Änderungen des Durchsatzes und der Prozessparameter der Herstellung bzw. in unterschiedlichen Produktionsanlagen auftreten. Wünschenswert ist eine Aciditätsschwankung unter 5%. Dies würde dadurch erreicht, dass dem MDI je nach gegebener Acidität das aciditätsaktive Zusatzmittel in einer Menge zugesetzt wird, so dass die Acidität oberhalb des Maximalwertes der prozessbedingten Schwankungsbreite des jeweiligen MDI liegt. In diesem Beispiel läge der Maximalwert somit bei 100 ppm + 50 ppm = 150 ppm. Die technisch verfügbaren Einrichtungen zur Dosierung der sauren Verbindung sind so exakt, dass die dann erreichte Schwankungsbreite vernachlässigbar gering ist. Analog stellt sich der Fall beim MMDI dar mit einer ungefähren Acidität von z.B. 10 ppm mit Schwankungen von bis zu 100 %. Hier wird in analoger Weise die saure Verbindung als aciditätsaktives Additiv zum MMDI zugesetzt.

In Schritt e) werden die in Schritt c) erhaltene Fraktion enthaltend mindestens 5 Gew.-% 3-Kern- und höherkerniges MDI und/oder die Fraktion enthaltend mindestens 95 Gew.-% 2-Kern-MDI mit einer sauren Verbindung versetzt, so dass die gewünschte Acidität erreicht wird.

Hierzu kann gasförmiges HCl benutzt werden. Da das Handhaben von HCl als Gas bei der Dosierung apparativ aufwendig ist, werden vorzugsweise flüssige oder feste organische Verbindungen verwendet die den Aciditätswert erhöhen, wie z.B. Benzoylchlorid, Phthalsäuredichlorid, Terephthalsäuredichlorid, Isophthalsäuredichloride, p-Toluolsulfonsäurechlorid, oder andere organische Säurechloride. Geeignet sind auch organische Säuren die den Aciditätswert erhöhen, wie z.B. Chlorpropionsäure, Bevorzugt werden dabei organische Verbindungen eingesetzt, die sich gut in MDI lösen und zugleich einen niedrigen Dampfdruck aufweisen und die sonstigen Eigenschaften der gewünschten Isocyanat-Additionsprodukte im Fertigteil nicht ändern.

Als anorganische saure Verbindungen sind geeignet wasserfreie Mineralsäuren wie z.B. HCl-Gas oder andere Halogenwasserstoffe, Phosphorsäure oder andere phosphorhaltige wasserfreie Säuren, Schwefelsäure, und andere Mineralsäuren. Anorganische wasserfreie Lewis-Säuren wie z.B. Aluminiumtrichlorid, Bortrifluorid, Bortrichlorid sind ebenfalls geeignet.

Geeignete organische saure Verbindungen, die den Aciditätswert erhöhen sind aliphatische oder cycloaliphatische Carbonsäurehalogenide wie beispielsweise Essigsäurechlorid, Trichloressigsäurechlorid oder N-Chloracetamid oder N-Bromsuccinimid, aromatische Carbonsäurehalogenide wie beispielsweise Benzoylchlorid, Phthalsäurechlorid, Terephthalsäuredichlorid, Isophthtalsäuredichlorid, aromatische, aliphatische oder cycloaliphatische Carbaminsäurehalogenide, insbesondere Carbaminsäurechloride wie beispielsweise N-Phenylcarbamylchlorid, tert.-Butylcarbamylchlorid, saure Chlor-Silanverbindungen wie beispielsweise Trimethylsilylchlorid, Trimethylsilyltrifluoromethansulfonat, Sulfonsäurehalogenide wie beispielsweise Tosylchlorid, oder weitere Carbonsäurederivate wie beispielsweise Anhydride oder Gemische daraus.

Bevorzugt werden Benzoylchlorid, Phthalsäuredichlorid, Terephthalsäuredichlorid, Butyl-carbamoylchloride, Trimethylsilyltrifluoromethansulfonat eingesetzt

Vorzugsweise werden bei der Einstellung der Acidität für MDI und den MDI-Mischungen mit 2-Kern-MDI-Gehalten von größer 80 Gew.-% und deren Derivaten und Modifizierungen, wie Allophanaten, Carbodiimiden, Uretoniminen, Uretdionen und prepolymeren Urethanen und/oder prepolymeren Harnstoffen aromatische bzw. aliphatische Säurechloride oder Carbamylchloride eingesetzt. Bevorzugt werden dabei aromatische, bzw. aliphatischen Säurechloride oder Carbamylchloride eingesetzt, die bei der Herstellung der Polyurethane nicht stören und einen niedrigen Dampfdruck aufweisen. Bevorzugt eingesetzt werden Benzoylchlorid, Terephthalsäuredichlorid, Isophthalsäuredichlorid, Phenylcarbamylchlorid, tert. Butylcarbamylchlorid.

Die Einbringung der sauren Verbindung kann direkt in das gewünschte MDI erfolgen oder in Form einer höher konzentrierten Stammlösung, die bevorzugt mit dem entsprechenden MDI angesetzt wird, das die Spezifikation des gewünschten MDI's nicht ändert.

Durch das erfindungsgemäße Verfahren wird erreicht, dass das erhaltene MDI eine definierte Acidität aufweist, und daher ein konstantes und definiertes Reaktionsverhalten gegenüber H-aktiven Verbindungen ergibt. Dies wirkt sich positiv auf eine störungsfreie Verarbeitung bei der Serienfertigung von daraus hergestellten Polyadditions Fertigteilen aus.

Im erfindungsgemäßen Verfahren werden zusätzlich zu den sauren anorganischen oder organischen Verbindungen oder deren Gemischen in Schritt e) noch inerte Übergangsmetallverbindungen, nämlich Eisen-Verbindungen, in Form von anorganischen oder organischen Eisen-Salzen oder Eisen-Komplexen zugegeben, die die Reaktion der IsocyanatGruppen mit der H-aciden-Verbindung bei der späteren Polyurethan-Herstellung beschleunigen. Das PMDI und das MMDI können aufgrund des Herstellprozesses, bedingt durch Korrosion und Erosion der Anlagenteile, geringe Spurenanteile an Eisen enthalten, die stark streuen können und dann zu schwankenden Reaktivitäten führen. Die Streuungen in der Reaktivität können dadurch ausgeglichen werden, dass ein konstanter Eisen-Spurengehalt durch Zusatz von entsprechenden Spurenanteilen an geeigneten Eisen-Verbindungen eingestellt wird.

Geeignete Eisen-Verbindungen zur Einstellung eines konstanten Eisen-Spurengehaltes sind anorganische oder organische Eisen-Salze von Mineralsäuren, von aliphatischen, alicyclischen und aromatischen Carbonsäuren und Alkoxiden oder Eisen-Komplexverbindungen. Die zugesetzten Eisen-Verbindungen müssen wasserfrei sein. Geeignete Eisen-Salze sind erfindungsgemäß Fe(III)-Chlorid, Fe (II)-Acetat, Fe (III)-Bromid, Fe (II)-Fluorid, Fe (II)-Iodid, Fe (II)-Fumarat, Fe (III)-tribenzoat, Fe (III)-trisalicylat, Fe (III)-trilaurat, Fe (III)-tripalmitat, Fe (III)-tristearat, Fe (III)-trioleat, Fe -Naphthenate, Fe (II) -2-Ethylhexanoat, Fe (II)-tris(4-cyclohexylbutyrat), Fe (III)-Ethoxid, Fe (III)-i-Propoxid. Geeignete Eisen-Komplexverbindungen sind beispielsweise Fe (III)-Acetylacetonat, Fe (III)-Benzoylacetonat, Fe (II)-Phthalocyanin, Ferrocen, Fe (III)-Trifluoracetylacetonat. Bevorzugt eingesetzt werden Fe (III)-Chlorid und Fe (III)-Acetylacetonat.

Darüber hinaus können auch in der PU-Verarbeitung bekannte Metall-Katalysatoren wie beispielsweise, Zinkchlorid, Dibutylzinndilaurat (DBTDL) Zinnoctoat oder andere organische Zinnverbindungen zugegeben werden, um die Reaktivität für bestimmte Anwendungen definiert einstellen zu können. Die Zugabe erfolgt hierbei in dem Maße, dass die spezifizierte Lagerstabilität von i.d.R. 6 Monaten weiterhin gewährleistet werden kann.

Die nach dem erfindungsgemäßen Verfahren hergestellten MDI-Produkte mit definiertem Wert für die Acidität können mit einem oder mehreren Gemischen enthaltend aromatische Isocyanate abgemischt werden. Für die Abmischung können neben den bereits erwähnten, bevorzugt eingesetzten Gemischen enthaltend Di- und/oder Polyisocyanate der Diphenylmethanreihe bevorzugt auch Gemische enthaltend Toluylendiisocyanat (zum Beispiel 2,4-TDI, 2,6-TDI) oder Gemische enthaltend Naphthalindiisocyanat (zum Beispiel 1,5-NDI) oder Gemische aus diesen Isocyanaten eingesetzt werden. Grundsätzlich können für die Abmischung aber auch andere aromatische und/oder aliphatische Mono-, Di-, Tri- oder höherfunktionellen Isocyanate eingesetzt werden.

Die nach dem erfindungsgemäßen Verfahren hergestellten MDI Produkte mit definiertem Werten für die Acidität können zu den üblichen Modifizierungsreaktionen von Isocyanaten benutzt werden, wie z.B. zur Carbodiimidisierung durch Temperaturerhöhung oder Zugabe von Phospholenoxiden und zur auf den Carbodiimiden aufbauenden Bildung von Uretoniminen, oder zur Herstellung von Prepolymeren aus OH-funktionellen Polyestern, C₂/C₃/C₄-Polyethern, Uretdionen, Allophanaten, Biureten oder Harnstoffderivaten.

Die nach dem erfindungsgemäßen Verfahren hergestellten Fraktionen von Diisocyanaten der Diphenylmethenreihe finden Verwendung unter anderem als Abmischkomponenten für andere Di- und Polyisocyanate der MDI-Reihe und/oder anderen Isocyanaten zur Herstellung von Isocyanatkomponenten für den Einsatz in der Herstellung von Polyurethanenen. Die aus diesen Abmischprodukten herzustellenden Fertigteile decken das gesamte Feld der Polyurethan-Chemie ab. Beispielhaft genannt sind hier:
- Hartschaumstoffe für die Isolier und Kühlgeräteindustrie
- Verpackungsschaum
- Weichschaum und Weichformschaum für Möbel und Autoindustrie
- Anwendungen im Spektrum der CASE-Anwendungen (Coatings, Adhesives, Sealants, Elastomers)
- Halbharte PU-Schaumstoffe

### Beispiele:

### Beispiel 1 (Einstellung der Acidität eines polymeren MDI (PMDI) - nicht gemäß Patentanspruch 1):

Es werden 11 Partien eines polymeren MDI nach dem erfindungsgemäßen Verfahren nach den Schritten a) bis c) hergestellt. Die 11 Partien weisen ein Gehalt an 3-Kern- und höherkernigem MDI von 46-49 Gew.-% auf.

In Schritt d) wird die Acidität der einzelnen Partien analytisch bestimmt. Die Bestimmung der Acidität erfolgt dabei nach folgender Methode:
Etwa 6 g der MDI-Polymer-Probe wurden auf mindestens 0,01 g genau in ein 250 ml-Becherglas eingewogen. Dann wurden 60ml Chlorbenzol zugegeben und die MDI-Probe in der zugebenen Menge Chlorbenzol gelöst. Die Lösung wurde anschließend mit 100 ml Methanol versetzt, das Becherglas abgedeckt und das Gemisch auf einem Magnetrührer 30 min bei Raumtemperatur gerührt. Abschließend wurde das Gemisch mit einer methanolischen Kaliumhydroxid-Maßlösung mit einer Stoffmengenkonzentration von ca. 0,01 mol/l acidimetrisch unter potentiometrischer Endpunktanzeige titriert.

Bei der Analyse ergibt sich, dass die einzelnen Partien im Ausgangszustand eine Acidität von 41,6 + / - 6,2 mg H⁺ / kg mit einer relativen Standardabweichung von 14,9 % aufweisen.

Anschließend werden in Schritt e) die 11 Partien des polymeren MDI mit Chlorwasserstoff-Gas auf eine Acidität von 100 mg/kg (gerechnet als H⁺, normierte Werte) eingestellt. Dazu wird für jede einzelne Partie die für die Einstellung der Acidität von 100 mg H⁺ / kg erforderliche Menge an Chlorwasserstoff-Gas zudosiert Nach der Einstellung ist die Streuung der Aciditätswerte deutlich geringer (100 + / - 3,5 mg H⁺ / kg, relative Standardabweichung 3,5 %).

### Beispiel 2 (Einstellung des Gehalts an hydrolysierbarem Chlor für monomeres MDI - nicht gemäß Patentanspruch 1)

Für monomeres MDI wird die Acidität des MDI als Wert für hydrolysierbares Chlor bestimmt.

Es werden 85 Partien eines monomeren MDI nach dem erfindungsgemäßen Verfahren nach den Schritten a) bis c) hergestellt. Die 85 Partien weisen ein Gehalt an 2-Kern-MDI von >98,2 Gew.-% auf. Die einzelnen 2-Kern-MDI-Isomere sind dabei in folgenden Mengen enthalten: 4,4'-MDI (≥ 98,0%), 2,4'-MDI (≤ 2,0%) und 2,2'-MDI (≤ 0,3%) bezogen auf die Summe der 2-Kern-MDI-Isomeren.

In Schritt d) wird der Wert für hydrolysierbares Chlor bestimmt. Die Bestimmung des Werts für hydrolysierbares Chlor erfolgt dabei nach folgender Methode:
Etwa 10 g der MDI-Monomer-Probe wurden in einem 500 ml-Erlenmeyerkolben eingewogen. Dann wurde das MDI-Monomer in siedenden Wasserbad aufgeschmolzen und in 40 ml Chlorbenzol gelöst. Anschließend wurden 100 ml Propanol-(2) zugegeben, der Erlenmeyer-Kolben abgedeckt und das Reaktionsgemisch auf einem Magnetrührer 5 min bei Raumtemperatur gerührt. Dann wurden 100 ml Methanol zugegeben und das Gemisch weitere 5 min bei Raumtemperatur gerührt. Anschließend wurden 100 ml Wasser zugegeben, ein Rückflusskühler aufgesetzt und das Reaktionsgemisch 30 min unter leichtem Rühren zum Sieden erhitzt. Nach Abkühlung wurden 50 ml Aceton durch den Rückflusskühler zugegeben Abschließend wurde der Chlorid-Gehalt des Gemisches durch argentometrische Titration mit einer Silbernitrat-Maßlösung mit einer Stoffmengenkonzentration von ca. 0,002 mol/l unter potentiometrischer Endpunktanzeige titriert.

Bei der Analyse ergibt sich, dass die einzelnen Partien einen Gehalt an hydrolysierbarem Chlor aufweisen, der für die einzelnen Partien stark streut. Der Gehalt an hydrolysierbarem Chlor beträgt für die 85 Partien 27,4 +/- 5,3 mg HCl / kg mit einer relativen Standardabweichung von 19,3 %.

Anschließend werden in Schritt e) die 85 Partien des monomeren MDI durch Zugabe von Isophthalsäuredichlorid auf einen Gehalt an hydrolysierbarem Chlor von 100 mg/kg (gerechnet als HCl, normierte Werte) eingestellt. Dazu wird für jede einzelne Partie die für die Einstellung auf einen Wert für hydrolysierbares Chlor von 100 mg HCl/kg erforderliche Menge an Isophthalsäuredichlorid zudosiert. Dabei erfolgt die Zugabe des Isophthalsäuredichlorids als Masterbatch, gelöst in einem Gemisch aus 45 % 4,4'-MDI und 55 % 2.,4'-MDI. Nach der Einstellung ist die Streuung der Gehalte an hydrolysierbarem Chlor deutlich geringer (100 +/- 3,3 mg HCl / kg; relative Standardabweichung 3,3 %).

## Patentansprüche

1. Verfahren zur Herstellung von Di- und Polyisocyanaten der Diphenylmethanreihe (MDI) und
Gemischen daraus mit definiertem und konstantem Wert für den Säuregehalt (Acidität), bei dem man
a) Anilin und Formaldehyd in Gegenwart eines sauren Katalysators zu Di- und Polyaminen der Diphenylmethanreihe umsetzt, und
b) die Di- und Polyamine der Diphenylmethanreihe gegebenenfalls in Gegenwart eines Lösungsmittels phosgeniert, wobei man ein rohes Di- und Polyisocyanat erhält, und
c) aus dem rohen Di- und Polyisocyanat destillativ wenigstens eine Fraktion enthaltend mindestens 5 Gew.-% 3-Kern- und höherkerniges MDI sowie wenigstens eine Fraktion enthaltend mindestens 95 Gew.-% 2-Kern-MDI abtrennt, und
d) die Acidität der Fraktion enthaltend mindestens 5 Gew.-% 3-Kern- und höherkerniges MDI analytisch bestimmt, und
e) die Fraktion enthaltend mindestens 5 Gew.-% 3-Kern- und höherkerniges MDI mit einer sauren Verbindung versetzt, so dass die gewünschte Acidität gezielt eingestellt wird, wobei zusätzlich eine inerte Übergangsmetallverbindung enthaltend Eisen zugesetzt wird,
wobei die saure Verbindung ausgewählt ist aus organischen Säuren, wasserfreien Mineralsäuren, anorganischen wasserfreien Lewis-Säuren, aliphatischen und/oder aromatischen und/oder cycloaliphatischen Carbonsäurehalogeniden, aliphatischen und/oder aromatischen und/oder cycloaliphatischen Carbaminsäurehalogeniden, sauren Chlor-Silanverbindungen, Sulfonsäurehalogeniden und Carbonsäureanhydriden,
und wobei die inerte Übergangsmetallverbindung enthaltend Eisen wasserfrei ist und ausgewählt ist aus Fe (III)-Chlorid, Fe (II)-Acetat, Fe (III)-Bromid, Fe (II)-Fluorid, Fe (II)-Iodid, Fe (II)-Fumarat, Fe (III)-tribenzoat, Fe (III)-trisalicylat, Fe (III)-trilaurat, Fe (III)-tripalmitat, Fe (III)-tristearat, Fe (III)-trioleat, Fe - Naphthenate, Fe (II)-2-Ethylhexanoat, Fe (II)-tris(4-cyclohexylbutyrat), Fe (III)-Ethoxid, Fe (III)-i-Propoxid, Fe (III)-Acetylacetonat, Fe (III)-Benzoylacetonat, Fe (II)-Phthalocyanin, Ferrocen und Fe (III)-Trifluoracetylacetonat.

2. Verfahren nach Anspruch 1, bei dem die inerte Übergangsmetallverbindung Eisenacetylacetonat oder Eisen (III)-Chlorid ist.

## Claims

1. Process for the production of di- and polyisocyanates of the diphenylmethane series (MDI) and mixtures thereof having a defined and constant value for the acid content (acidity), in which
a) aniline and formaldehyde are reacted in the presence of an acid catalyst to form di- and polyamines of the diphenylmethane series, and
b) the di- and polyamines of the diphenylmethane series are phosgenated, optionally in the presence of a solvent, to obtain a crude di- and polyisocyanate, and
c) from the crude di- and polyisocyanate, at least one fraction containing at least 5 wt.% 3-ring and multi-ring MDI and at least one fraction containing at least 95 wt.% 2-ring MDI are separated by distillation, and
d) the acidity of the fraction containing at least 5 wt.% 3-ring and multi-ring MDI is determined analytically, and
e) an acidic compound is added to the fraction containing at least 5 wt.% 3-ring and multi-ring MDI so that the desired acidity is achieved, wherein an inert transition metal compound containing iron is additionally added,
wherein the acidic compound is selected from organic acids, anhydrous mineral acids, inorganic anhydrous Lewis acids, aliphatic and/or aromatic and/or cycloaliphatic carbonyl halides, aliphatic and/or aromatic and/or cycloaliphatic carbamoyl halides, acidic chlorosilane compounds, sulphonyl halides and carboxylic anhydrides,
and wherein the inert transition metal compound containing iron is anhydrous and is selected from Fe(III) chloride, Fe(II) acetate, Fe(III) bromide, Fe(II) fluoride, Fe(II) iodide, Fe(II) fumarate, Fe(III) tribenzoate, Fe(III) trisalicylate, Fe(III) trilaurate, Fe(III) tripalmitate, Fe(III) tristearate, Fe(III) trioleate, Fe naphthenates, Fe(II) 2-ethylhexanoate, Fe(II) tris(4-cyclohexylbutyrate), Fe(III) ethoxide, Fe(III) i-propoxide, Fe(III) acetylacetonate, Fe(III) benzoylacetonate, Fe(II) phthalocyanine, ferrocene and Fe(III) trifluoroacetylacetonate.

2. Process according to claim 1, wherein the inert transition metal compound is iron acetylacetonate or iron(III) chloride.

## Revendications

1. Procédé de fabrication de di- et polyisocyanates de la série du diphénylméthane (MDI) et leurs mélanges ayant une valeur définie et constante pour la teneur en acide (acidité), selon lequel
a) de l'aniline et du formaldéhyde sont mis en réaction en présence d'un catalyseur acide pour former des di- et polyamines de la série du diphénylméthane, et
b) les di- et polyamines de la série du diphénylméthane sont éventuellement phosgénées en présence d'un solvant, un di- et polyisocyanate brut étant obtenu, et
c) au moins une fraction contenant au moins 5 % en poids de MDI à 3 noyaux et davantage, ainsi qu'au moins une fraction contenant au moins 95 % en poids de MDI à 2 noyaux sont séparées du di- et polyisocyanate brut par distillation, et
d) l'acidité de la fraction contenant au moins 5 % en poids de MDI à 3 noyaux et davantage est déterminée analytiquement, et
e) la fraction contenant au moins 5 % en poids de MDI à 3 noyaux et davantage est mélangée avec un composé acide, afin d'ajuster l'acidité souhaitée de manière ciblée, un composé de métal de transition inerte contenant du fer étant ajouté en outre,
le composé acide étant choisi parmi les acides organiques, les acides minéraux anhydrides, les acides de Lewis anhydres inorganiques, les halogénures d'acides carboxyliques aliphatiques et/ou aromatiques et/ou cycloaliphatiques, les halogénures d'acides carbamiques aliphatiques et/ou aromatiques et/ou cycloaliphatiques, les composés de chlorosilane acides, les halogénures d'acide sulfonique et les anhydrides d'acides carboxyliques,
et le composé de métal de transition inerte contenant du fer étant anhydre et étant choisi parmi le chlorure de Fe(III), l'acétate de Fe(II), le bromure de Fe(III), le fluorure de Fe(II), l'iodure de Fe(II), le fumarate de Fe(II), le tribenzoate de Fe(III), le trisalicylate de Fe(III), le trilaurate de Fe(III), le tripalmitate de Fe(III), le tristéarate de Fe(III), le trioléate de Fe(III), le naphténate de Fe, le 2-éthylhexanoate de Fe(II), le tris(4-cyclohexylbutyrate) de Fe(II), l'éthoxyde de Fe(III), l'i-propoxyde de Fe(III), l'acétylacétonate de Fe(III), le benzoylacétonate de Fe(III), la phtalocyanine de Fe(II), le ferrocène et le trifluoroacétylacétonate de Fe(III).

2. Procédé selon la revendication 1, selon lequel le composé de métal de transition inerte est l'acétylacétonate de fer ou le chlorure de fer (III).
